# EUROPEAN PATENT SPECIFICATION

(11) **EP 0 921 787 B1**
(45) Date of publication and mention of the grant of the patent: **29.10.2003**
(21) Application number: 97930933.3
(22) Date of filing: 18.06.1997
(51) Int. Cl.: A61K 9/00, A61K 31/44

(54) **ADMINISTRATION REGIMEN OF H+,K+-ATPase INHIBITORS**
PLAN ZUR VERABREICHUNG VON H+, K+-ATPase INHIBITOREN
REGIME D'ADMINISTRATION DES INHIBITEURS DE H+,K+-ATPase

(30) Priority: 20.06.1996 SE 9602442
(43) Date of publication of application: 16.06.1999
(73) Proprietor: AstraZeneca AB, 151 85 Södertälje (SE)
(72) Inventor: CEDERBERG, Christer, S-431 69 Mölndal (SE); SACHS, George, Encino, CA 91316 (US)
(86) International application number: SE9701098
(87) International publication number: WO97048380

(56) References cited:
- WO-A-96/01623
- US-A- 4 853 230
- JOHN E. HOOVER, "Remington's Pharmaceutical Sciences", 1975, MACK PUBLISHING COMPANY, PENNSYLVANIA, page 702.

## Description

### Field of the invention

The present invention is related to a new administration regimen of proton pump inhibitors, i.e. H⁺, K⁺-ATPase inhibitors. The new administration regimen gives an extended blood plasma concentration profile of the pharmaceutical substance, i.e. the proton pump inhibitors, thereby giving an improved inhibition of gastric acid secretion and an improved therapeutic effect. More specifically, the invention refers to the use of pharmaceutical preparations with a controlled release in the treatment of gastric acid-related diseases. The pharmaceutical preparation is preferably in the form of a dosage form which provides an extended and constant release of the acid labile H⁺, K⁺-ATPase inhibitor in the small and/or large intestines (but not in stomach) or a dosage form which provides two or more discrete pulses of release of the H⁺,K⁺-ATPase inhibitor in the small and/or large intestines (but not in stomach) separated in time with 0.5 - 4 hours. Furthermore, the present invention refers to the manufacture of such preparations.

### Background of the invention

Acid labile H⁺, K⁺-ATPase inhibitors also named as gastric proton pump inhibitors are for instance compounds known under the generic names omeprazole, lansoprazole, pantoprazole, pariprazole and leminoprazole. Some of these compounds are for instance disclosed in EP-A1-0005129, WO 94/27988, EP-A1-174726, EP-A1-166287 and GB 2163747.

These pharmaceutical substances are useful for inhibiting gastric acid secretion in mammals including man by controlling gastric acid secretion at the final step of the acid secretory pathway and thus reduce basal and stimulated gastric acid secretion irrespective of stimulus. In a more general sense, they may be used for prevention and treatment of gastric-acid related diseases in mammals and man, including e.g. reflux oesophagitis, gastritis, duodenitis, gastric ulcer, duodenal ulcer and Zollinger-Ellison syndrom. Furthermore, they may be used for treatment of other gastrointestinal disorders where gastric acid inhibitory effect is desirable e.g. in patients on NSAID therapy, in patients with Non Ulcer Dyspepsia, and in patients with symptomatic gastro-esophageal reflux disease. They may also be used in patients in intensive care situations, in patients with acute upper gastrointestinal bleeding, pre-and postoperatively to prevent aspiration of gastric acid and to prevent and treat stress ulceration. Further, they may be useful in the treatment of psoriasis as well as in the treatment of *Helicobacter* infections and diseases related to these.

Therapeutic control of gastric acid secretion is fundamental in all theses diseases, but the degree and duration of acid inhibition required for optimal clinical effect is not fully understood.

The duration of acid inhibition of one proton pump inhibitor such as for instance omeprazole is 3 - 4 days despite a plasma half-life of only 0.5 - 1 hour (Lind et al, Gut 1983;24:270-276)). This lack of temporal relationship between plasma concentration of omeprazole and the degree of acid inhibition is due to the long-lasting binding of the active inhibitor to the gastric pump.

Proton pump inhibitors, such as the above discussed omeprazole, are generally administered as a single daily dose of 20 mg to 40 mg, depending on the gastrointestinal disorder as well as the severity of the disease. In the treatment of Zollinger-Ellison syndrom higher dosages of 60 - 120 mg/daily and as much as 360 mg/daily have been used. Generally, the proton pump inhibitor is adminstered to the patient during 2 - 4 weeks, in some cases up to 8 weeks. Omeprazole has also been used as maintainace therapy for peptic ulcer disease and reflux oesophagitis during many years.

Despite this long duration of acid inhibition once daily dosing results in not more than 70-80 % inhibition of maximal acid output prior to next dose. Results from *Helicobacter pylori* eradication studies have shown an improved efficacy with twice daily dosing in combination with antimicrobials. Treatment of severe GORD is also improved by divided doses as compared to single daily dose increments. These improved clinical effects are due to longer periods of high acid inhibition.

Although action of proton pump inhibitors is covalent, efficacy depends on active pumps and there are two pools of pumps, active and inactive. Only active pumps are covalently inhibited. The inactive pumps are recruited throughout the day therefore effectiveness of acid inhibition improves for 72 hours on once a day treatment, steady state being achieved as a balance between inhibition of active pumps and *de novo* biosynthesis or reversal of inhibition.

Extended release formulations to give blood plasma levels extending from 6-12 hours (by any of several means) will result in a larger fraction of the pumps being inhibited and should result in more effective inhibition of acid secretion resulting in improved efficacy in GORD, more rapid healing of gastric ulcer and improved eradication of *H*. *Pylori.*

### Detailed description of the drawings

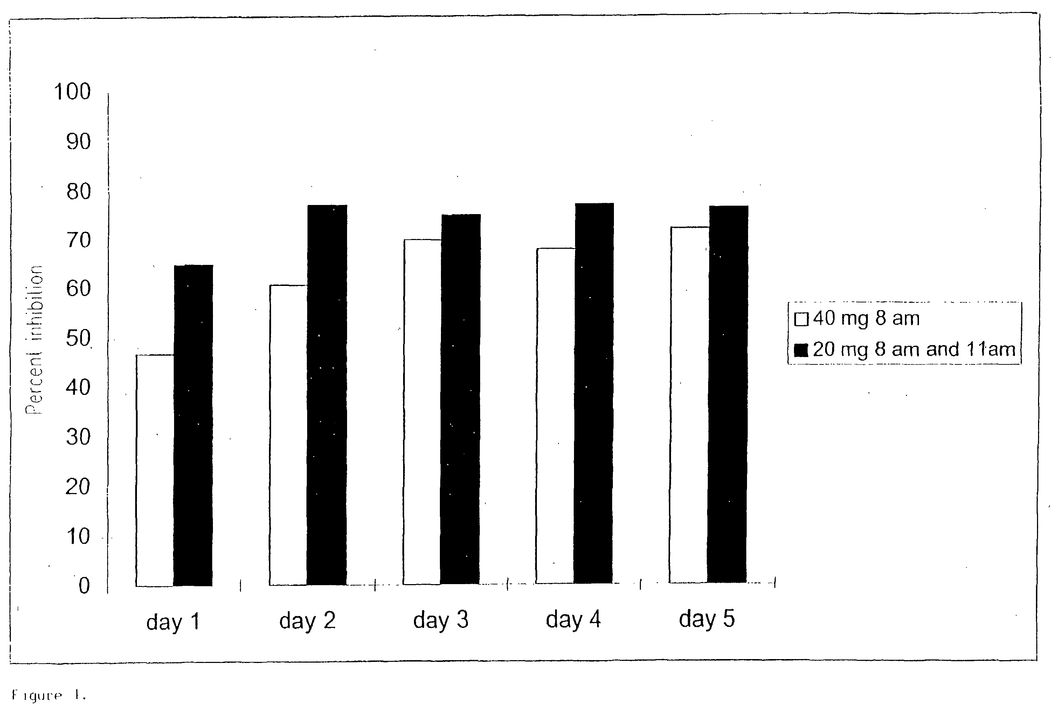

Figure 1 shows two graphs. These show the differencies between once daily administration and administration of two consecutive doses within 3 hours.

### Summary of the invention

The invention is as defined in the claims.

On a once a day administration regimen the maximal effect of omeprazole is about 75 % to 80 %, 24 hours after dose (Lind et al 1986, Scand J Gastroenterol (Suppl 118): 137 - 8 and Lind et al 1988, Scand J Gastroenterol 23: 1259 - 66), i.e. about 20 % to 25 % of the maximal gastric acid secretory capacity is present 24 hours after the dose. Even if an increased dose quantity of the proton pump inhibitor has been used (See Lind et al) the maximal gastric acid inhibition is limited to about 80 %.

The known dose dependency of gastric acid inhibition has hithereto resulted in a recommendation to initially increase the dose of the proton pump inhibitor, if a low response on the therapy or lack of response is obtained.

It has now been proposed according to the present invention to extend the plasma concentration profile of proton pump inhibitors and thereby improving their therapeutic effect. According to one aspect of the invention the extended plasma profile is provided by once daily administration of a dosage form which releases the proton pump inhibitor with an almost constant rate during an extended time period. According to another aspect of the invention the extended plasma profile is provided by once daily administration of a dosage form which, in the small and/or large intestines (but not in the stomach), releases the proton pump inhibitor in discrete pulses separated in time by 0.5 - 4 hours. It is also possible to obtain an extended plasma profile of a proton pump inhibitor by consecutive administrations of two or more unit doses with 0.5 - 4 hours intervals.

### Detailed description of the invention

Acid secretion by the gastric mucosa is a property of the parietal cell. Whereas the functional regulation of this cell is a complicated process involving several different cell types with different receptors, acid transport *per se* is the property of a single P-type ATPase, the gastric H⁺, K⁺-ATPase. Therefore, effective therapeutic control of acid secretion involves either receptor blockade or gastric H⁺, K⁺-ATPase inhibition. This invention relates to the proton pump inhibitors and their reaction with the gastric acid pump. The half-life in plasma of the proton pump inhibitors is rather short. The administered proton pump inhibitor reacts with the active gastric acid pumps available for inhibition during that time. Un-inhibited, inactive pumps will be present during this time and pumps will recover following biosynthesis and reversal of inhibition. Therefore, by a repeated regimen or a dosage form which-provides an extended plasma profile of the proton pump inhibitors recovered pumps as well as un-inhibited pumps not previously available will react with the newly administered dose or pulse of pharmaceutical substance or the continuously released substance.

By administration of a pharmaceutical dosage form with an extended release, the plasma concentration of the pharmaceutical substance can be kept on a high level during an extended time. As a result the number of pumps inhibited by the proton pump inhibitor will increase and a more efficient therapeutic control of acid secretion will be obtained.

Compounds of interest for the novel administration with a repeated dosing regimen as well as for the controlled release preparations/compositions giving an extended plasma profile according to the present invention are compounds of the general formula I 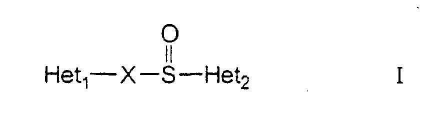 wherein
Het₁ is 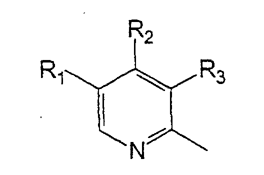 or 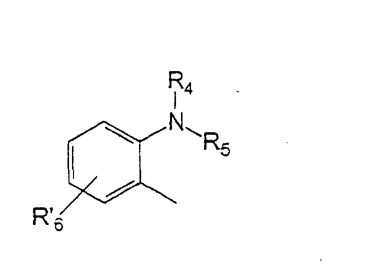 Het₂ is 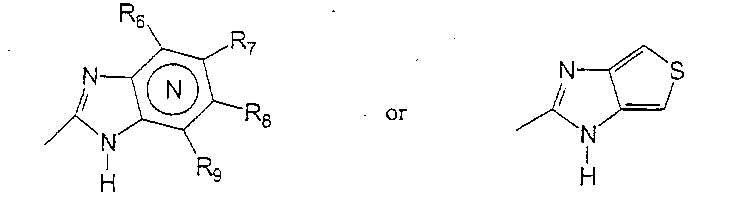 X= 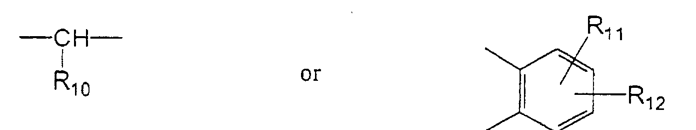 wherein
N in the benzimidazole moiety means that one of the ring carbon atoms substituted by R₆-R₉ optionally may be exchanged for a nitrogen atom without any substituents;
R₁, R₂ and R₃ are the same or different and selected from hydrogen, alkyl, alkoxy optionally substituted by fluorine, alkylthio, alkoxyalkoxy, dialkylamino, piperidino, morpholino, halogen, phenyl and phenylalkoxy;
R₄ and R₅ are the same or different and selected from hydrogen, alkyl and aralkyl;
R₆' is hydrogen, halogen, trifluoromethyl, alkyl and alkoxy;
R₆-R₉ are the same or different and selected from hydrogen, alkyl, alkoxy, halogen, haloalkoxy, alkylcarbonyl, alkoxycarbonyl, oxazolyl, trifluoroalkyl, or adjacent groups R₆-R₉ form ring structures which may be further substituted;
R₁₀ is hydrogen or forms an alkylene chain together with R₃ and
R₁₁ and R₁₂ are the same or different and selected from hydrogen, halogen or alkyl.

Examples of specifically interesting compounds according to formula I are

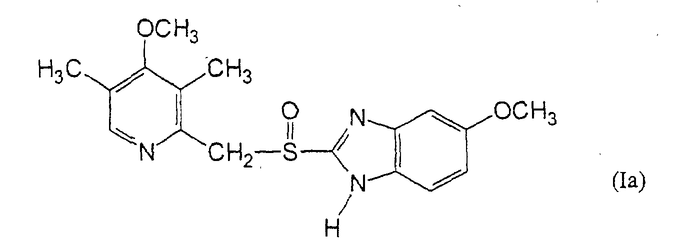

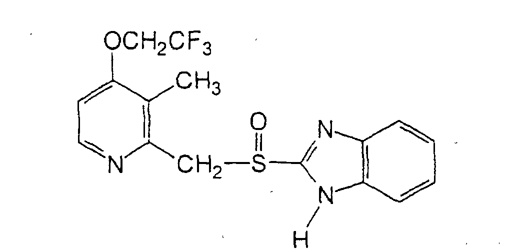

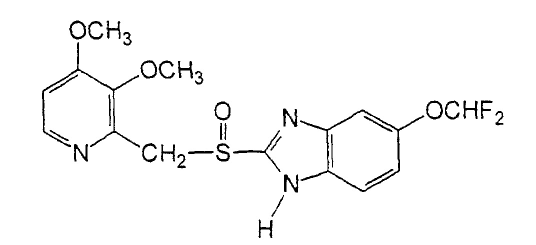

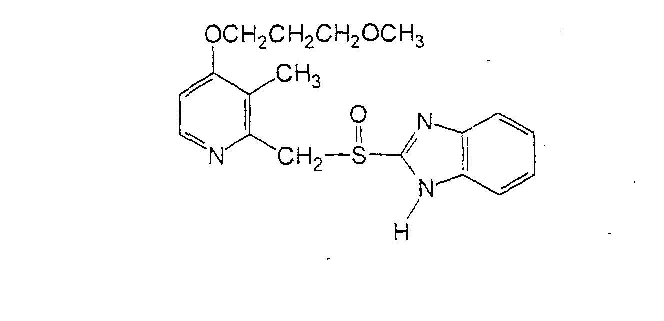

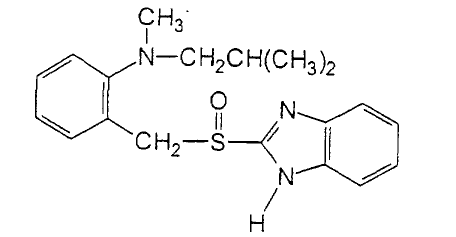

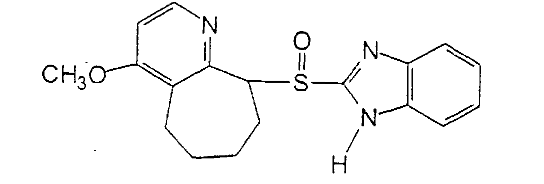

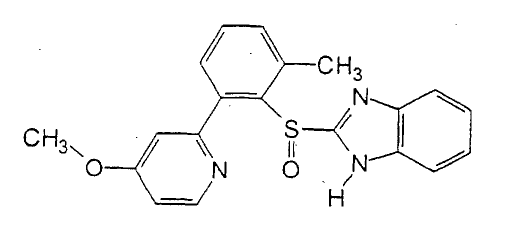

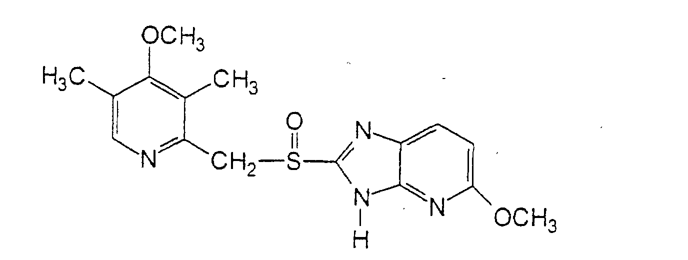

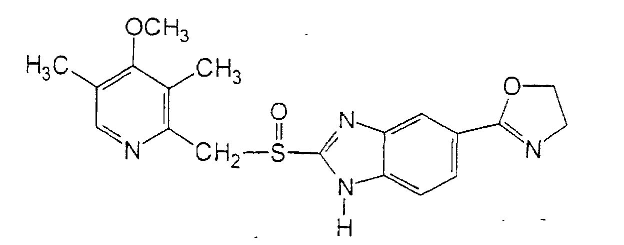

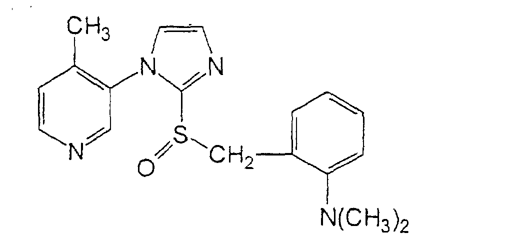

The compound used in the administration regimen as well as in the controlled release preparations according to the present invention may be used in neutral form or in the form of an alkaline salt, such as for instance the Mg²⁺, Ca²⁺, Na⁺ or K⁺ salts, preferably the

Mg²⁺ salts. The compounds may also be used in the form of one of its single enantiomers or an alkaline salt of the single enantiomer.

Preferred compounds for the administration regimen and the oral pharmaceutical preparation according to the present invention are omeprazole, a magnesium salt of omeprazole or a magnesium salt of the (-)-enantiomer of omeprazole.

The above compounds are susceptible to degradation/transformation in acidic and neutral media. Generally, the degradation is catalyzed by acidic reacting compounds and the active compounds are stabilized with alkaline reacting compounds. Thus, the substances being acid labile proton pump inhibitors are best protected from contact with acidic gastric juice by an enteric coating. There are different enteric coating layered preparations comprising omeprazole as well as other proton pump inhibitors described in the prior art, see for instance US-A 4,853,230. An enteric coated tablet of omeprazole magnesium salt is described in WO 95/ 01783. A tableted multiple unit dosage form of omeprazole is described in WO 96/ 01623. Pharmaceutical preparations manufactured according to known principles as described in the specifications US-A 4,853,230, WO 95/ 01783 and WO 96/ 01623, may be used for administration with an increased dosing frequency according to the present invention.

A unit dosage of the proton pump inhibitor, for instance 1 - 500 mg is administered at least twice a day. The unit dosage may be given with a dosing frequency of about 0.5 - 4 hours, preferably two doses are given during a time period of 2 to 3 hours. Suitable doses comprise for instance 5, 10, 15, 20, 30 and 40 mg of the pharmaceutical substance.

In another embodiment of the invention an extended plasma profile is obtained by administration of a unit dose of a proton pump inhibitor which releases the drug for absorption in the small and/or large intestines in discrete pulses seperated in time by 0.5 - 4 hours.

Alternatively, an oral pharmaceutical formulation with extended release of the pharmaceutical substance during 2 - 12 hours, preferably 4 - 8 hours may be administered. Such an extended release preparation may comprise up to 500 mg of the substance, preferably the doses comprise about 5 - 100 mg of the substance, and more preferably 10 - 80 mg.

Different techniques for manufacturing of various controlled release preparations are for example described in Aulton M.E. (Churchill Livingstone Ed.), Pharmaceutics: The science of dosage form design (1988), p. 316-321.

The invention is described more in detail by the following examples.

### Examples

Omeprazole (Prilosec® capsules) 40 mg once daily (adminstered at 8.00 a.m.) or 20 mg given twice daily (adminstered at 8.00 a.m. and at 11.00 a.m.) given during five consecutive days were compared regarding effect on peptone stimulated gastric acid secretion and intragastric acidity measured on days 1 to 3 and day 5 in eigth healthy subjects. During the first two days of treatment there was a significantly (p>0.05) lower number of hours with high acidity (pH>1) when omeprazole was given twice daily, 20 mg administered with 3 hours apart, compared to a single morning dose of 40 mg. There was also a significantly higher degree of hihibition of peptone stimulated acid output 24 hours post dose during the first three days of treatment. See Figure 1. These results clearly support the concept of extended plasma profiles of orneprazole being beneficial in optimising control of acid secretion.

## Claims

1. Use of a H⁺, K⁺-ATPase inhibitor for the manufacturing of a medicament for oral administration such that an extended blood plasma profile is obtained by administration of two or more consecutive oral unit doses of said H⁺, K⁺-ATPase inhibitor with 0.5 - 4 hours intervals for an improved inhibition of gastric acid secretion, which H⁺, K⁺-ATPase inhibitor is a compound with formula I 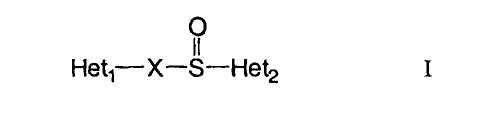 wherein
Het₁ is 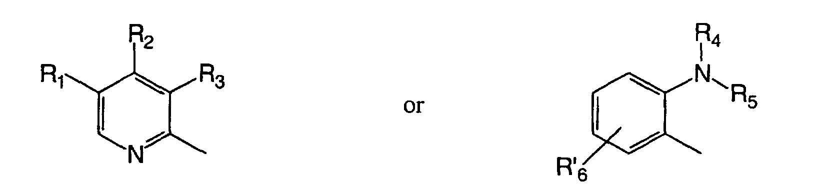 Het₂ is 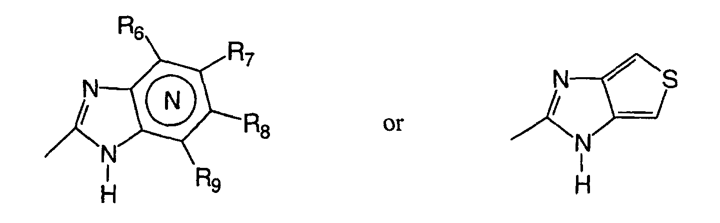 X= 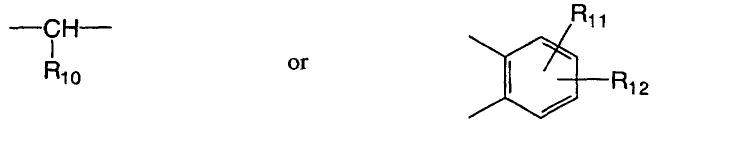 wherein
N in the benzimidazole moiety means that one of the ring carbon atoms substituted by R₆-R₉ optionally may be exchanged for a nitrogen atom without any substituents;
R₁, R₂ and R₃ are the same or different and selected from hydrogen, alkyl, alkoxy optionally substituted by fluorine, alkylthio, alkoxyalkoxy, dialkylamino, piperidino, morpholino, halogen, phenyl and phenylalkoxy;
R₄ and R₅ are the same or different and selected from hydrogen, alkyl and aralkyl;
R'₆ is hydrogen, halogen, trifluoromethyl, alkyl and alkoxy;
R₆-R₉ are the same or different and selected from hydrogen, alkyl, alkoxy, halogen, halo-alkoxy, alkylcarbonyl, alkoxycarbonyl, oxazolyl, trifluoroalkyl, or adjacent groups R₆-R₉ form ring structures which may be further substituted;
R₁₀ is hydrogen or forms an alkylene chain together with R₃ and
R₁₁ and R₁₂ are the same or different and selected from hydrogen, halogen or alkyl.

2. Use of a H⁺, K⁺-ATPase inhibitor for manufacturing of a medicament for oral administration such that an extended blood plasma profile is obtained by administration of two or more consecutive oral unit doses of said H⁺, K⁺-ATPase inhibitor with 0.5 - 4 hours intervals for an improved therapeutic effect in the treatment of gastrointestinal disorders associated with excess gastric acid secretion, which H⁺, K⁺-ATPase inhibitor is a compound with formula I defined in claim 1.

3. Use of a H⁺, K⁺-ATPase inhibitor for manufacturing of a medicament for oral administration according to claim 1 or 2 **characterized in that** the extended blood plasma profile is obtained by administration of a pharmaceutical preparation which release the medicament for absorption in two or more discrete pulses separated in time by 0.5 - 4 hours intervals, which H⁺; K⁺-ATPase inhibitor is a compound with formula I defined in claim 1.

4. Use according to any of claims 1 - 3 **characterized in that** the extended plasma profile is obtained by oral administration of a unit dose of a pharmaceutical preparation which releases the H⁺, K⁺-ATPase inhibitor for absorption with an almost constant rate during an extended time period.

5. Use according to any of claims 1 - 4 **characterized in that** the extended plasma profile is received during 2 - 12 hours.

6. Use according to any of claims 1 - 5 **characterized in that** the H⁺, K⁺-ATPase inhibitor is a compound selected from the group of omeprazole, an alkaline salt of omeprazole, the (-)-enantiomer of omeprazole and an alkaline salt of the (-)-enantiomer of omeprazole.

## Patentansprüche

1. Verwendung eines H⁺,K⁺-ATPase-Inhibitors zur Herstellung eines Arzneimittels zur derartigen oralen Verabreichung, daß durch Verabreichung einer oder mehrerer aufeinanderfolgender oraler Einheitsdosen des H⁺,K⁺-ATPase-Inhibitors in einem zeitlichen Abstand von 0,5 bis 4 Stunden ein verlängertes Blutplasmaprofil zwecks verbesserter Inhibierung der Magensäuresekretion erhalten wird, wobei es sich bei dem H⁺,K⁺-ATPase-Inhibitor um eine Verbindung der Formel I 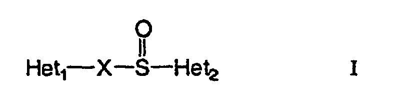 worin
Het₁ für 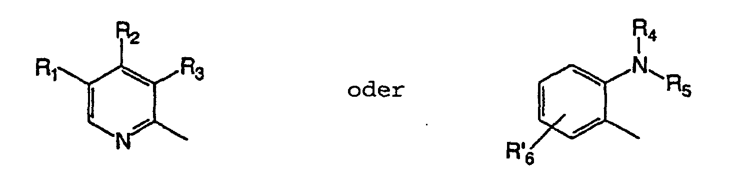 steht,
Het₂ für 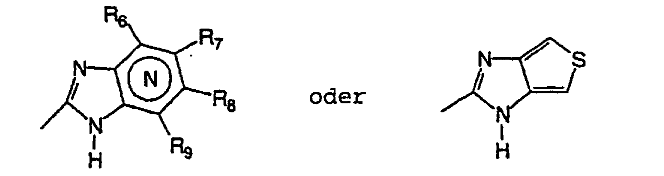 steht,
X = 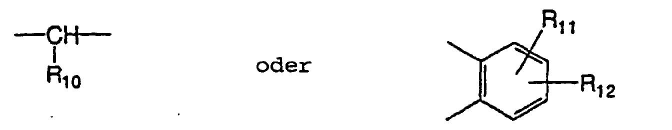 worin
N in der Bezimidazolgruppierung bedeutet, daß eines der durch R₆-R₉ substituierten Ringkohlenstoffatome gegebenenfalls durch ein Stickstoffatom ohne Substituenten ersetzt sein kann;
R₁, R₂ und R₃ gleich oder verschieden sind und aus der Gruppe bestehend aus Wasserstoff, Alkyl, gegebenenfalls durch Fluor substituiertes Alkoxy, Alkylthio, Alkoxyalkoxy, Dialkylamino, Piperidino, Morpholino, Halogen, Phenyl und Phenylalkoxy ausgewählt sind;
R₄ und R₅ gleich oder verschieden sind und aus der Gruppe bestehend aus Wasserstoff, Alkyl und Aralkyl ausgewählt sind;
R₆' für Wasserstoff, Halogen, Trifluormethyl, Alkyl oder Alkoxy steht;
R₆-R₉ gleich oder verschieden sind und aus der Gruppe bestehend aus Wasserstoff, Alkyl, Alkoxy, Halogen, Halogenalkoxy, Alkylcarbonyl, Alkoxycarbonyl, Oxazolyl und Trifluoralkyl ausgewählt sind oder benachbarte Gruppen R₆-R₉ gegebenenfalls weiter substituierte Ringstrukturen bilden;
R₁₀ für Wasserstoff steht oder gemeinsam mit R₃ eine Alkylenkette bildet und
R₁₁ und R₁₂ gleich oder verschieden sind und aus der Gruppe bestehend aus Wasserstoff, Halogen oder Alkyl ausgewählt sind; handelt.

2. Verwendung eines H⁺,K⁺-ATPase-Inhibitors zur Herstellung eines Arzneimittels zur derartigen oralen Verabreichung, daß durch Verabreichung einer oder mehrerer aufeinanderfolgender oraler Einheitsdosen des H⁺,K⁺-ATPase-Inhibitors in einem zeitlichen Abstand von 0,5 bis 4 Stunden ein verlängertes Blutplasmaprofil zwecks verbesserter therapeutischer Wirkung bei der Behandlung von mit übermäßiger Magensäuresekretion einhergehenden gastrointestinalen Störungen erhalten wird, wobei es sich bei dem H⁺,K⁺-ATPase-Inhibitor um eine Verbindung der Formel I gemäß der in Anspruch 1 angegebenen Definition handelt.

3. Verwendung eines H⁺,K⁺-ATPase-Inhibitors zur Herstellung eines Arzneimittels zur oralen Verabreichung nach Anspruch 1 oder 2, **dadurch gekennzeichnet, daß** das verlängerte Blutplasmaprofil dadurch erhalten wird, daß man eine pharmazeutische Zubereitung verabreicht, die das Arzneimittel zur Resorption in zwei oder mehr diskreten Pulsen mit einem zeitlichen Abstand von 0,5 bis 4 Stunden freisetzt, wobei es sich bei dem H⁺,K⁺-ATPase-Inhibitor um eine Verbindung der Formel I gemäß der in Anspruch 1 angegebenen Definition handelt.

4. Verwendung nach einem der Ansprüche 1 bis 3, **dadurch gekennzeichnet, daß** das verlängerte Blutplasmaprofil dadurch erhalten wird, daß man eine pharmazeutische Zubereitung verabreicht, die den H⁺,K⁺-ATPase-Inhibitor zur Resorption mit fast konstanter Rate über einen längeren Zeitraum freisetzt.

5. Verwendung nach einem der Ansprüche 1 bis 4, **dadurch gekennzeichnet, daß** das verlängerte Blutplasmaprofil über einen Zeitraum von 2 bis 12 Stunden empfangen wird.

6. Verwendung nach einem der Ansprüche 1 bis 5, **dadurch gekennzeichnet, daß** es sich bei dem H⁺,K⁺-ATPase-Inhibitor um eine Verbindung aus der Gruppe bestehend aus Omeprazol, einem alkalischen Salz von Omeprazol, dem (-)-Enantiomer von Omeprazol und einem alkalischen Salz des (-)-Enantiomers von Omeprazol handelt.

## Revendications

1. Utilisation d'un inhibiteur de H⁺,K⁺-ATPase pour la fabrication d'un médicament destiné à être administré par voie orale de sorte qu'un profil de plasma sanguin prolongé soit obtenu par l'administration de deux ou plusieurs doses unitaires orales consécutives dudit inhibiteur de H⁺,K⁺-ATPase avec 0,5 - 4 heures d'intervalles pour une meilleure inhibition de la sécrétion d'acide gastrique, lequel inhibiteur de H⁺,K⁺-ATPase est un composé de formule I 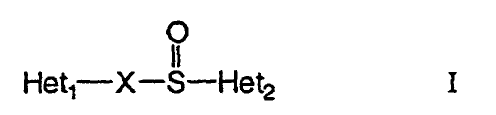 dans laquelle
Het₁ est 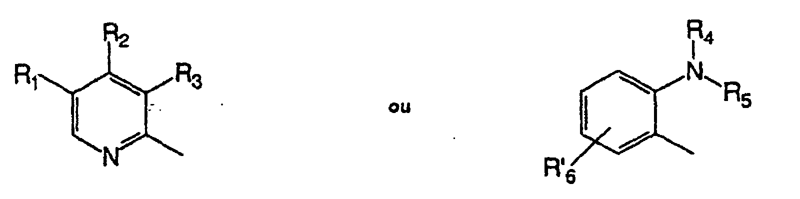 Het₂ est 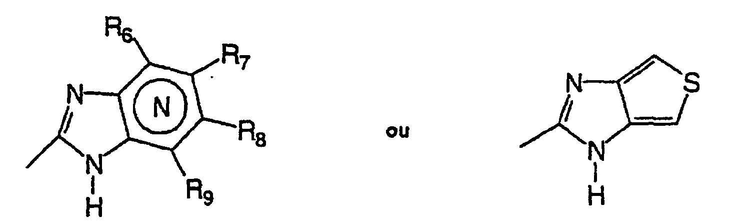 X = 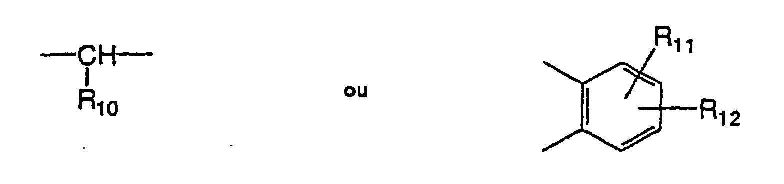 où
N dans le motif benzimidazole signifie que l'un des atomes de carbone du cycle substitués par R₆-R₉ peut être éventuellement échangé pour un atome d'azote sans aucun substituant ;
R₁, R₂ et R₃ sont identiques ou différents et sont choisis parmi hydrogène, alkyle, alcoxy éventuellement substitué par fluor, alkylthio, alcoxyalcoxy, diakylamino, pipéridino, morpholino, halogène, phényle et phénylalcoxy ;
R₄ et R₅ sont identiques ou différents et sont choisis parmi hydrogène, alkyle et aralkyle ;
R₆' est hydrogène, halogène, trifluorométhyle, alkyle et alcoxy ;
R₆-R₉ sont identiques ou différents et sont choisis parmi hydrogène, alkyle, alcoxy, halogène, halogénoalcoxy, alkylcarbonyle, alcoxycarbonyle, oxazolyle, trifluoroalkyle, ou des groupements adjacents R₆-R₉ forment des structures en cycle pouvant être encore substituées ;
R₁₀ est hydrogène ou forme une chaîne alkylène conjointement avec R₃ et
R₁₁ et R₁₂ sont identiques ou différents et sont choisis parmi hydrogène, halogéno ou alkyle.

2. Utilisation d'un inhibiteur de H⁺,K⁺-ATPase pour la fabrication d'un médicament destiné à être administré par voie orale de sorte qu'un profil de plasma sanguin prolongé soit obtenu par l'administration de deux ou plusieurs doses unitaires orales consécutives dudit inhibiteur de H⁺,K⁺-ATPase avec 0,5 - 4 heures d'intervalles pour un meilleur effet thérapeutique dans le traitement de troubles gastro-intestinaux associés à un excès de sécrétion d'acide gastrique, lequel inhibiteur de H⁺,K⁺-ATPase est un composé de formule I définie dans la revendication 1.

3. Utilisation d'un inhibiteur de H⁺,K⁺-ATPase pour la fabrication d'un médicament destiné à l'administration par voie orale selon la revendication 1 ou 2, **caractérisée en ce que** le profil de plasma sanguin prolongé est obtenu par administration d'une préparation pharmaceutique libérant le médicament pour une absorption en deux ou plusieurs quantités discrètes pulsées séparées dans le temps par 0,5 - 4 heures d'intervalles, lequel inhibiteur de H⁺,K⁺-ATPase est un composé de formule I définie dans la revendication 1.

4. Utilisation selon l'une quelconque des revendications 1 à 3, **caractérisée en ce que** le profil de plasma prolongé est obtenu par l'administration par voie orale d'une dose unitaire d'une préparation pharmaceutique libérant l'inhibiteur de H⁺,K⁺-ATPase pour l'absorption à une vitesse presque constante au cours d'une période de temps prolongée.

5. Utilisation selon l'une quelconque des revendications 1 - 4, **caractérisée en ce que** le profil de plasma prolongé est reçu pendant 2 - 12 heures.

6. Utilisation selon l'une quelconque des revendications 1 - 5, **caractérisée en ce que** l'inhibiteur de H⁺,K⁺-ATPase est un composé choisi dans le groupe d'oméprazole, d'un sel alcalin d'oméprazole, l'énantiomère (-) d'oméprazole et un sel alcalin de l'énantiomère (-) d'oméprazole.
